(19) Europäisches Patentamt
European Patent Office
Office européen des brevets

(11) EP 2 348 038 A1

(12) EUROPÄISCHE PATENTANMELDUNG

(43) Veröffentlichungstag:
**27.07.2011 Patentblatt 2011/30**

(51) Int Cl.:
***C07K 7/06*** *(2006.01)* ***C12Q 1/52*** *(2006.01)*
***G01N 33/58*** *(2006.01)*

(21) Anmeldenummer: **10151487.5**

(22) Anmeldetag: **22.01.2010**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA RS**

(71) Anmelder: **PHILIPPS-UNIVERSITÄT MARBURG
35037 Marburg (DE)**

(72) Erfinder:
- **Steinmetzer, Torsten
07743 Jena (DE)**
- **Becker, Gero
35037 Marburg (DE)**

(74) Vertreter: **Stumpf, Peter
Kerkrader Straße 3
35394 Gießen (DE)**

(54) **Verfahren zur Bestimmung der Aktivität der Transglutaminase Faktor XIIIa**

(57) Die vorliegende Erfindung betrifft ein UV/VIS- oder fluoreszenzbasierendes Verfahren zur qualitativen und quantitativen Bestimmung der Aktivität des Blutgerinnungsfaktors XIIIa, bzw. des Gehalts an Zymogen Faktor XIII, welches zu Faktor XIIIa aktiviert wird, unter Verwendung neuer chromogener oder fluoreszenzmarkierter Peptidsubstrate sowie die Verwendung solcher Verfahren und/oder Substrate in Screeningassays.

**Fig. 1**

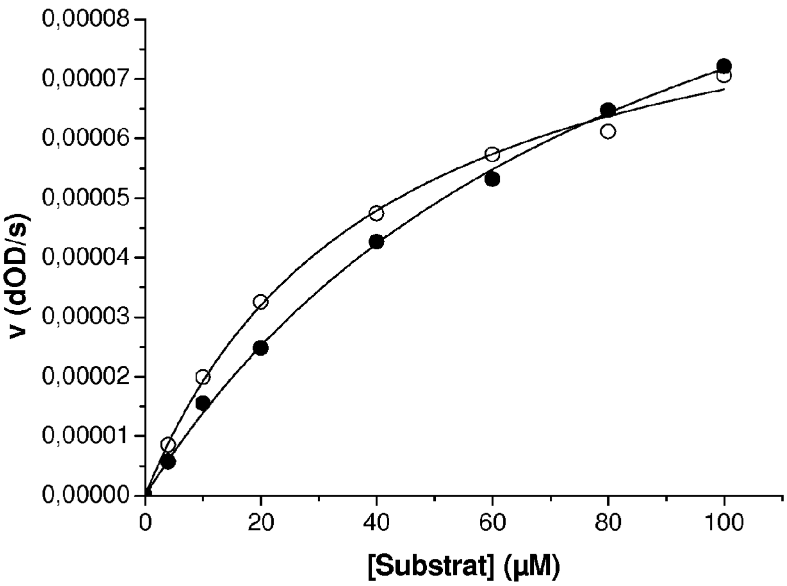


EP 2 348 038 A1

SEQUENCE LISTING

<110> Philipps-Universität Marburg

<120> Verfahren zur Bestimmung der Aktivität der Transglutaminase Faktor XIIIa

<130> TM405

<160> 22

<170> PatentIn version 3.5

<210> 1
<211> 8
<212> PRT
<213> Artificial Sequence

<220>
<223> Xaa represents a glutamic acid residue wherein the amino group on carbon atom No. 5 is replaced by a p-nitroanilide group (pNA).

<220>
<221> MISC_FEATURE
<222> (1)..(8)
<223> Xaa represents a glutamic acid residue wherein the amino group on carbon atom No. 5 is replaced by a p-nitroanilide group (pNA).

<400> 1

Asn Xaa Glu Asn Val Ser Pro Leu
1               5

<210> 2
<211> 7
<212> PRT
<213> Artificial Sequence

<220>
<223> Xaa represents a glutamic acid residue wherein the amino group on carbon atom No. 5 is replaced by a p-nitroanilide group (pNA).

<220>
<221> MISC_FEATURE
<222> (1)..(7)
<223> Xaa represents a glutamic acid residue wherein the amino group on carbon atom No. 5 is replaced by a p-nitroanilide group (pNA).

<400> 2

Gly Xaa Ser Lys Val Ile Gly
1               5

<210> 3
<211> 7
<212> PRT
<213> Artificial Sequence

<220>
<223> Xaa represents a glutamic acid residue wherein the amino group on carbon atom No. 5 is replaced by a p-nitroanilide group (pNA).

<220>

<221> MISC_FEATURE
<222> (1)..(7)
<223> Xaa represents a glutamic acid residue wherein the amino group on carbon atom No. 5 is replaced by a p-nitroanilide group (pNA).

<400> 3

Pro Xaa Lys Lys Val Ile Gly
1 5

<210> 4
<211> 7
<212> PRT
<213> Artificial Sequence

<220>
<223> Xaa represents a glutamic acid residue wherein the amino group on carbon atom No. 5 is replaced by a p-nitroanilide group (pNA).

<220>
<221> MISC_FEATURE
<222> (1)..(7)
<223> Xaa represents a glutamic acid residue wherein the amino group on carbon atom No. 5 is replaced by a p-nitroanilide group (pNA).

<400> 4

Tyr Xaa Ser Lys Val Ile Gly
1 5

<210> 5
<211> 7
<212> PRT
<213> Artificial Sequence

<220>
<223> Xaa represents a glutamic acid residue wherein the amino group on carbon atom No. 5 is replaced by a p-nitroanilide group (pNA).

<220>
<221> MISC_FEATURE
<222> (1)..(7)
<223> Xaa represents a glutamic acid residue wherein the amino group on carbon atom No. 5 is replaced by a p-nitroanilide group (pNA).

<400> 5

Ile Xaa Val Lys Val Ile Gly
1 5

<210> 6
<211> 7
<212> PRT
<213> Artificial Sequence

<220>
<223> Xaa represents a glutamic acid residue wherein the amino group on carbon atom No. 5 is replaced by a p-nitroanilide group (pNA).

<220>
<221> MISC_FEATURE
<222> (1)..(7)

<223> Xaa represents a glutamic acid residue wherein the amino group on carbon atom No. 5 is replaced by a p-nitroanilide group (pNA).

<400> 6

Ala Xaa Val Lys Val Ile Gly
1 5

<210> 7
<211> 7
<212> PRT
<213> Artificial Sequence

<220>
<223> Xaa represents a glutamic acid residue wherein the amino group on carbon atom No. 5 is replaced by a p-nitroanilide group (pNA).

<220>
<221> MISC_FEATURE
<222> (1)..(7)
<223> Xaa represents a glutamic acid residue wherein the amino group on carbon atom No. 5 is replaced by a p-nitroanilide group (pNA).

<400> 7

Phe Xaa Val Lys Val Ile Gly
1 5

<210> 8
<211> 7
<212> PRT
<213> Artificial Sequence

<220>
<223> Xaa1: bound to H and Xaa2, represents a D-phenylalanine (dPhe) residue. Xaa2: bound to Xaa1 and Val, represents a Glu residue wherein the NH2 group on C-5 is replaced by p-nitroanilide (pNA).

<220>
<221> MISC_FEATURE
<222> (1)..(7)
<223> Xaa1: bound to H and Xaa2, represents a D-phenylalanine (dPhe) residue. Xaa2: bound to Xaa1 and Val, represents a Glu residue wherein the NH2 group on C-5 is replaced by p-nitroanilide (pNA).

<400> 8

Xaa Xaa Val Lys Val Ile Gly
1 5

<210> 9
<211> 7
<212> PRT
<213> Artificial Sequence

<220>
<223> Xaa represents a glutamic acid residue wherein the amino group on carbon atom No. 5 is replaced by a p-nitroanilide group (pNA).

<220>
<221> MISC_FEATURE
<222> (1)..(7)

<223> Xaa represents a glutamic acid residue wherein the amino group on carbon atom No. 5 is replaced by a p-nitroanilide group (pNA).

<400> 9

Tyr Xaa Lys Lys Val Ile Gly
1 5

<210> 10
<211> 7
<212> PRT
<213> Artificial Sequence

<220>
<223> Xaa represents a glutamic acid residue wherein the amino group on carbon atom No. 5 is replaced by a p-nitroanilide group (pNA).

<220>
<221> MISC_FEATURE
<222> (1)..(7)
<223> Xaa represents a glutamic acid residue wherein the amino group on carbon atom No. 5 is replaced by a p-nitroanilide group (pNA).

<400> 10

Tyr Xaa Val Lys Val Ile Gly
1 5

<210> 11
<211> 7
<212> PRT
<213> Artificial Sequence

<220>
<223> Xaa represents a glutamic acid residue wherein the amino group on carbon atom No. 5 is replaced by a p-nitroanilide group (pNA).

<220>
<221> MISC_FEATURE
<222> (1)..(7)
<223> Xaa represents a glutamic acid residue wherein the amino group on carbon atom No. 5 is replaced by a p-nitroanilide group (pNA).

<400> 11

Tyr Xaa Lys Val Val Ile Gly
1 5

<210> 12
<211> 6
<212> PRT
<213> Artificial Sequence

<220>
<223> Xaa represents a glutamic acid residue wherein the amino group on carbon atom No. 5 is replaced by a p-nitroanilide group (pNA).

<220>
<221> MISC_FEATURE
<222> (1)..(6)
<223> Xaa represents a glutamic acid residue wherein the amino group on carbon atom No. 5 is replaced by a p-nitroanilide group (pNA).

<400> 12

```
Tyr Xaa Val Lys Val Ile
1               5
```

<210> 13
<211> 5
<212> PRT
<213> Artificial Sequence

<220>
<223> Xaa represents a glutamic acid residue wherein the amino group on carbon atom No. 5 is replaced by a p-nitroanilide group (pNA).

<220>
<221> MISC_FEATURE
<222> (1)..(5)
<223> Xaa represents a glutamic acid residue wherein the amino group on carbon atom No. 5 is replaced by a p-nitroanilide group (pNA).

<400> 13

```
Tyr Xaa Val Lys Val
1               5
```

<210> 14
<211> 7
<212> PRT
<213> Artificial Sequence

<220>
<223> Xaa represents a glutamic acid residue wherein the amino group on carbon atom No. 5 is replaced by a p-nitroanilide group (pNA).

<220>
<221> MISC_FEATURE
<222> (1)..(7)
<223> Xaa represents a glutamic acid residue wherein the amino group on carbon atom No. 5 is replaced by a p-nitroanilide group (pNA).

<400> 14

```
Tyr Xaa Ile Lys Val Ile Gly
1               5
```

<210> 15
<211> 7
<212> PRT
<213> Artificial Sequence

<220>
<223> Xaa represents a glutamic acid residue wherein the amino group on carbon atom No. 5 is replaced by a p-nitroanilide group (pNA).

<220>
<221> MISC_FEATURE
<222> (1)..(7)
<223> Xaa represents a glutamic acid residue wherein the amino group on carbon atom No. 5 is replaced by a p-nitroanilide group (pNA).

<400> 15

```
Tyr Xaa Leu Lys Val Ile Gly
1               5


<210>  16
<211>  7
<212>  PRT
<213>  Artificial Sequence

<220>
<223>  Xaa represents a glutamic acid residue wherein the amino group on
       carbon atom No. 5 is replaced by a p-nitroanilide group (pNA).


<220>
<221>  MISC_FEATURE
<222>  (1)..(7)
<223>  Xaa represents a glutamic acid residue wherein the amino group on
       carbon atom No. 5 is replaced by a p-nitroanilide group (pNA).

<400>  16

Tyr Xaa Val Arg Val Ile Gly
1               5


<210>  17
<211>  6
<212>  PRT
<213>  Artificial Sequence

<220>
<223>  Xaa represents a glutamic acid residue wherein the amino group on
       carbon atom No. 5 is replaced by a p-nitroanilide group (pNA).


<220>
<221>  MISC_FEATURE
<222>  (1)..(6)
<223>  Xaa represents a glutamic acid residue wherein the amino group on
       carbon atom No. 5 is replaced by a p-nitroanilide group (pNA).

<400>  17

Tyr Xaa Val Lys Val Phe
1               5


<210>  18
<211>  7
<212>  PRT
<213>  Artificial Sequence

<220>
<223>  Xaa represents a glutamic acid residue wherein the amino group on
       carbon atom No. 5 is replaced by a p-nitroanilide group (pNA).


<220>
<221>  MISC_FEATURE
<222>  (1)..(7)
<223>  Xaa represents a glutamic acid residue wherein the amino group on
       carbon atom No. 5 is replaced by a p-nitroanilide group (pNA).

<400>  18

Lys Xaa Val Lys Val Ile Gly
```

1 5

<210> 19
<211> 7
<212> PRT
<213> Artificial Sequence

<220>
<223> Xaa1: bound to His and Xaa2, represents a Glu residue wherein the NH2 group on C-5 is replaced by p-nitroanilide (pNA). Xaa2: bound to Xaa1 and Lys, represents a Lys residue wherein the NH2 group on C-6 is protected by Z (carboxybenzyl)

<220>
<221> MISC_FEATURE
<222> (1)..(7)
<223> Xaa1: bound to His and Xaa2, represents a Glu residue wherein the NH2 group on C-5 is replaced by p-nitroanilide (pNA). Xaa2: bound to Xaa1 and Lys, represents a Lys residue wherein the NH2 group on C-6 is protected by Z (carboxybenzyl)

<400> 19

His Xaa Xaa Lys Val Ile Gly
1 5

<210> 20
<211> 7
<212> PRT
<213> Artificial Sequence

<220>
<223> Xaa1: bound to Tyr and Xaa2, represents a Glu residue wherein the NH2 group on C-5 is replaced by p-nitroanilide (pNA). Xaa2: bound to Xaa1 and Lys, represents a Lys residue wherein the NH2 group on C-6 is protected by Z (carboxybenzyl)

<220>
<221> MISC_FEATURE
<222> (1)..(7)
<223> Xaa1: bound to Tyr and Xaa2, represents a Glu residue wherein the NH2 group on C-5 is replaced by p-nitroanilide (pNA). Xaa2: bound to Xaa1 and Lys, represents a Lys residue wherein the NH2 group on C-6 is protected by Z (carboxybenzyl)

<400> 20

Tyr Xaa Xaa Lys Val Ile Gly
1 5

<210> 21
<211> 7
<212> PRT
<213> Artificial Sequence

<220>
<223> Xaa represents a glutamic acid residue wherein the amino group on carbon atom No. 5 is replaced by a 7-amido-5-methyl coumarin group (AMC)

<220>
<221> MISC_FEATURE

```
<222>  (1)..(7)
<223>  Xaa represents a glutamic acid residue wherein the amino group on
       carbon atom No. 5 is replaced by a 7-amido-5-methyl coumarin
       group (AMC)

<400>  21

Tyr Xaa Val Lys Val Ile Gly
1               5


<210>  22
<211>  7
<212>  PRT
<213>  Artificial Sequence

<220>
<223>  Xaa represents a glutamic acid residue wherein the amino group on
       carbon atom No. 5 is replaced by a 7-amido-5-methyl coumarin
       group (AMC)


<220>
<221>  MISC_FEATURE
<222>  (1)..(7)
<223>  Xaa represents a glutamic acid residue wherein the amino group on
       carbon atom No. 5 is replaced by a 7-amido-5-methyl coumarin
       group (AMC)

<400>  22

Tyr Xaa Val Arg Val Ile Gly
1               5
```

## Beschreibung

Die vorliegende Erfindung betrifft UV/VIS- oder fluoreszenzbasierende Verfahren zur qualitativen und quantitativen Bestimmung der Aktivität des Blutgerinnungsfaktors XIIIa, bzw. des Gehalts an Zymogen Faktor XIII, welcher zu Faktor XIIIa aktiviert werden kann. Dafür werden geeignete, neuartige chromogene oder fluoreszenzmarkierte Peptidsubstrate verwendet. Die Erfindung betrifft auch die Verwendung solcher Verfahren oder der Substrate für Screeningtests.

## Stand der Technik

Am Ende der Blutgerinnungskaskade führt die durch Thrombin katalysierte Abspaltung der Fibrinopeptide A und B zur Bildung von Fibrinpolymeren. Die Transglutaminase Faktor XIIIa (FXIIIa) katalysiert die dreidimensionale Quervernetzung der Fibrinpolymere durch die Ausbildung intermolekularer Amidbindungen zwischen Lysin- und Glutaminresten der $\gamma$- und $\alpha$-Ketten des Fibrins (Wilmer et al., Hämostaseologie 22 (2002) 18-28). Die Aktivierung des Zymogens FXIII zum FXIIIa erfolgt durch Thrombin in Gegenwart von $Ca^{2+}$-Ionen und Fibrin als akzelerierendem Kofaktor. Die Quervernetzung der Fibrinpolymere dient der mechanischen Stabilisierung des gebildeten Gerinnsels. Dessen Stabilität kann noch erhöht werden, indem weitere Proteine kovalent eingebaut werden oder indem das Gerinnsel an extrazelluläre Matrixbestandteile angekoppelt wird. Zusätzlich kann FXIIIa das Gerinnsel durch den Einbau antifibrinolytischer Faktoren, wie $\alpha_2$-Antiplasmin und Thrombin-aktivierbarem Fibrinolyseinhibitor vor vorzeitiger Lyse schützen.

Neben dem im Plasma vorkommenden Blutgerinnungsfaktor XIIIa, in der Literatur auch als pFXIIIa bezeichnet, gibt es noch einen zellulären FXIIIa, der oftmals mit cFXIIIa abgekürzt wird. Das Zymogen pFXIII ist ein tetramerer Komplex, der aus zwei A- und zwei B-Untereinheiten besteht, im Gegensatz dazu ist das Zymogen cFXIII nur ein Homodimer, das aus zwei A-Untereinheiten aufgebaut ist. Allerdings sind die Primärstruktur der A-Untereinheiten des pFXIII und cFXIII identisch. Nach thrombinkatalysierter Aktivierung des pFXIII (Abspaltung des Aktivierungspeptides durch Spaltung zwischen den Aminosäuren Arg37-Gly38 der A-Untereinheiten) kommt es in Gegenwart von $Ca^{2+}$ zur Dissoziation der beiden B-Untereinheiten aus dem tetrameren Komplex (Muszbek et al., Cardiovascular & Hematological Agents in Medicinal Chemistry 6 (2008) 190-205). Der aktivierte pFXIIIa ist deshalb identisch mit dem cFXIIIa, und darum gelten alle in dieser Patentanmeldung getroffenen Aussagen zu FXIII oder FXIIIa allgemein immer sowohl für pFXIII und pFXIIIa als auch für cFXIII und cFXIIIa.
Neben Blutungskomplikationen und Wundheilungsstörungen bei angeborenem oder erworbenen FXIII/FXIIIa-Mangel, spielt Faktor XIII/FXIIIa auch eine Rolle bei kardio- und zerebrovaskulären Erkrankungen. Es wird angenommen, dass sich durch die Entwicklung und Verwendung spezifischer Faktor XIIIa-Inhibitoren neue Therapiemöglichkeiten zur Behandlung thrombotischer Erkrankungen eröffnen (Prasa und Stürzebecher, Hämostaseologie 22 (2002) 29-33).

Für die Bestimmung der Konzentration des Faktors XIII bei Mangelzuständen und zur Aktivitätmessung des Faktors XIIIa bei thrombotischen Komplikationen oder bei der Entwicklung bzw. Verwendung von Hemmstoffen des Faktors FXIIIa ist ein einfaches Verfahren notwendig, dass eine zuverlässige, kostengünstige und automatisierbare Bestimmung des Faktors XIII bzw. des FXIIIa ermöglicht.

Die bisher bekannten Methoden zur Bestimmung des FXIII-Gehalts bzw. der FXIIIa-Aktivität lassen sich in verschiedene Gruppen einteilen: Semiquantitativ lässt sich die FXIIIa-Aktivität durch die Lysezeit bei der Gerinnselauflösung bestimmen, weiterhin kann man quantitative Methoden zur Ammoniakfreisetzung durch die FXIIIa-katalysierte Reaktion verwenden, ebenso ist es möglich den FXIIIakatalysierten Einbau primärer Amine zu untersuchen (siehe Übersichtsarbeit zu Methoden der Bestimmung des FXIII bzw. des FXIIIa: Wilmer et al. Hämostaseologie 22 (2002) 18-28) oder man kann die Aktivität des FXIIIa mit einem vor kurzem beschriebenen Fluoreszenztest bestimmen (Oertel et al., Analytical Biochemistry 367 (2007) 152-158).

Ein entscheidender Nachteil des Gerinnsel-Lyse-Tests ist die Tatsache, dass er nicht automatisierbar ist. Fickenscher et al. haben eine gekoppelten optischen Test für die Bestimmung der Faktor XIIIa-Aktivität entwickelt (Berichrom® FXIII) und verwenden dafür ein von der N-terminalen $\beta$-Caseinsequenz abgeleitetes Substrat (Leu-Gly-Pro-Gly-Gln-Ser-Lys-Val-Ile-Gly-Amid). Der durch die enzymatische Aktivität freigesetzte Ammoniak wird mittels einer Transaminasereaktion unter $NADH_2$-Verbrauch auf eine $\alpha$-Ketosäure übertragen, wobei der Verbrauch an $NADH_2$ bei 340 nm als Maß für die FXIIIa-Aktivität bestimmt wird (Fickenscher et al., Thromb. Haemost. 65, (1991) 535-540). Obwohl der Test prinzipiell automatisierbar ist, steht einer breiten Anwendung dieser photometrischen Methode derzeit die empfohlene Messwellenlänge von 340 nm entgegen, da der entsprechende Messkanal bei vielen Gerinnungsvollautomaten fehlt. Ein weiterer Nachteil ist eine existierende Lag-Phase, die zu einer nichtlinearen Kinetik führt, wodurch die Auswertung der Messung erschwert wird. Karpati et al. verwenden in einem analogen Test ein modifiziertes Substrat, das vom $\alpha_2$-Antiplasmin abgeleitet ist (Asn-Gln-Glu-Gln-Val-Ser-Pro-Ser-Thr-Leu-Leu-Lys) und konnten dadurch die Sensitivität des Tests um den Faktor 1,5 verbessern (Karpati et al., Clin. Chem. 46 (2000) 1946-1955).
Inkorporationstests nutzen die enzymatische Aktivität von FXIIIa als Endo-$\gamma$-Glutamin-$\varepsilon$-Lysin-Transferase, wobei die Bildung kovalenter Bindungen zwischen den Seitenketten der Aminosäuren Glutamin und Lysin ausgenutzt wird.

Während Glutamin für diesen Prozess essentiell ist, kann das Lysin durch eine Vielzahl anderer primärer Amine ersetzt werden. Es wurden verschiedene Verfahren entwickelt, mit denen man den Einbau des primären Amins direkt oder indirekt nachweisen kann (Zusammenfassung der Methoden in der Übersichtsarbeit: Wilmer et al., Hämostaseologie 22 (2002) 18-28). So wurden radioaktiv markierte Amine verwendet, allerdings eignet sich deren Verwendung nicht für den Routinenachweis. Zur Testautomatisierung wurden fluoreszenzmarkierte Amine genutzt und deren Einbau mittels Fluoreszenzpolarisation nachgewiesen. In einer weiteren Variation wurden biotinylierte Amine inkorporiert, deren Nachweis durch Streptavidin/alkalische Phosphatase-Konjugate möglich ist. Im Endeffekt wird die gekoppelte Phosphatase-Aktivität durch Umsetzung des Substrats p-Nitrophenylphosphat bestimmt. Allerdings ist der Test aufwendig und nur bedingt für die Routineanalyse einsetzbar.
Von Oertel et al. wurde ein fluorometrischer FXIIIa-Test entwickelt (Oertel et al., Analytical Biochemistry 367 (2007) 152-158). Dabei wird ein geeignetes Peptidsubstrat verwendet, dass N-terminal mit einem Fluoreszensdonor modifiziert ist, beispielsweise mit einem o-Aminobenzoylrest. Zusätzlich ist die Seitenkette des Glutamins in diesem Peptidsubstrat mit einem Spacer alkyliert, an den ein geeigneter Fluoreszenzlöscher gekoppelt ist, beispielsweise ein 2,4-Dinitrophenylrest. Durch die FXIIIa-katalysierte Spaltung der Glutamin-Seitenkettenamidgruppe wird die Fluoreszenzquenchung aufgehoben, dieser Vorgang ist mit einem Fluoreszenzspektrometer messbar. Die Synthese des modifizierten Dodecapeptids ist relativ aufwendig und kostspielig. Ein weiterer Nachteil besteht darin, dass viele der heutzutage standardmäßig eingesetzten Gerinnungsvollautomaten noch nicht für Fluoreszenzmessungen geeignet sind.

Nach derzeitigem Wissen existieren neben FXIII bzw. FXIIIa im Körper derzeit noch 8 weitere Transglutaminasen, dazu zählen TG1-TG7 sowie das Protein epb42, dass allerdings katalytisch unwirksam ist (Wodzinska, Mini Rev. in Med. Chem. 5 (2005) 279-292). Als erste Transglutaminase wurde das inzwischen als TG2 bezeichnete Enzym entdeckt. Dessen Aktivität lässt sich sehr einfach durch UV/VIS-spektroskopische oder fluorimetrische Messungen unter Verwendung simpler Aminosäurederiate nachweisen, beispielsweise durch Abspaltung von p-Nitroanlin aus H-Glu (pNA)-OH oder durch Abspaltung von Aminomethylcoumarin aus H-Glu(AMC)-OH (Smith et al., Anal. Biochem. 100 (1979) 136-139). Allerdings werden diese einfachen Derivate aufgrund anderer Substratspezifität von FXIIIa nicht gespalten. Bisher wurden auch noch keine Peptidderivate beschrieben, die einen Glu(pNA) oder Glu(AMC)-Rest besitzen und gleichzeitig mit ausreichender Effektivität von FXIIIa unter Freisetzung von pNA oder AMC umgesetzt werden. Initiale Versuche mit einem Glu(pNA)-haltigem Substrat, das sich von der Peptidsequenz des im Berichrom-Assay verwendeten Substrates ableitet, beispielsweise dem Peptid
H-Leu-Gly-Pro-Gly-Glu(pNA)-Ser-Lys-Val-Ile-Gly-$NH_2$ zeigten, dass es nur sehr langsam von FXIIIa gepalten wird und man nichtlineare Progresskurven erhält. Auch dessen verkürzte Derivate H-Gly-Pro-Gly-Glu(pNA)-Ser-Lys-Val-Ile-Gly-$NH_2$, H-Pro-Gly-Glu(pNA)-Ser-Lys-Val-Ile-Gly-$NH_2$, Ac-Glu(pNA)-Ser-Lys-Val-Ile-Gly-$NH_2$ oder H-Glu(pNA)-Ser-Lys-Val-Ile-Gly-$NH_2$ wurden fast gar nicht oder nur sehr langsam gepalten und eignen sich deshalb nicht als Substrate für ein routinemäßiges Verfahren zum Nachweis der FXIIIa-Aktivität.

## Aufgabe

Aufgabe der Erfindung ist es, ein verbessertes Verfahren zur Bestimmung der Aktivität von Transglutaminasen, speziell der Transglutaminase Faktor XIIIa, bereitzustellen.

## Lösung der Aufgabe

Überraschenderweise haben wir festgestellt, dass man die Aktivität des FXIIIa mittels standardmäßiger UV/VIS- oder Fluoreszenz-Verfahren effizient nachweisen kann, wenn man für diesen Zweck geeignete Peptidsubstrate entsprechend der allgemeinen Formel (I)

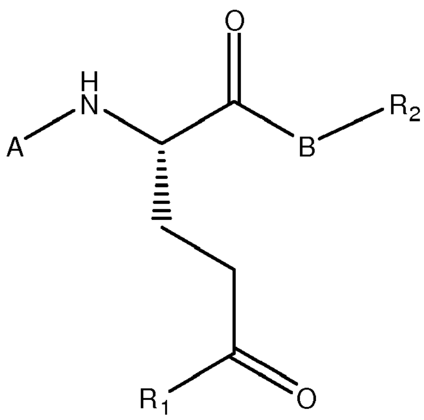

einsetzt, die ausgehend vom N-Terminus des Peptids als zweiten Aminosäurerest einen in der Seitenkette mit einer geeigneten Gruppe modifizierten Glutaminsäurerest besitzen, wobei diese geeignete Gruppe

- $R_1$ eine der folgenden Strukturen ist

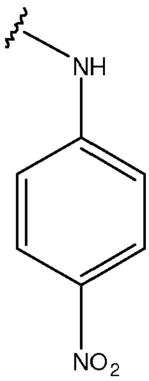
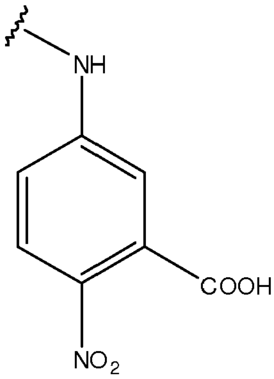
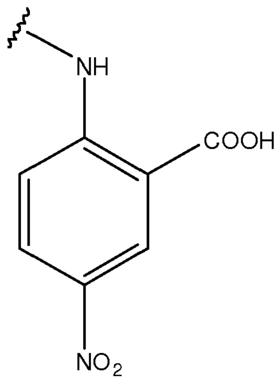
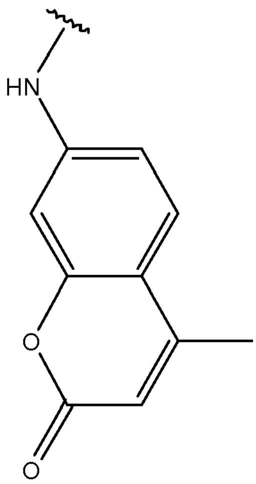

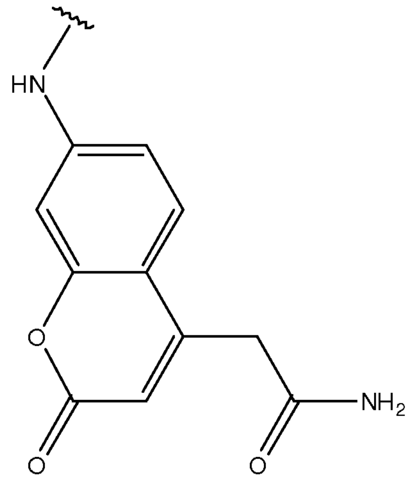

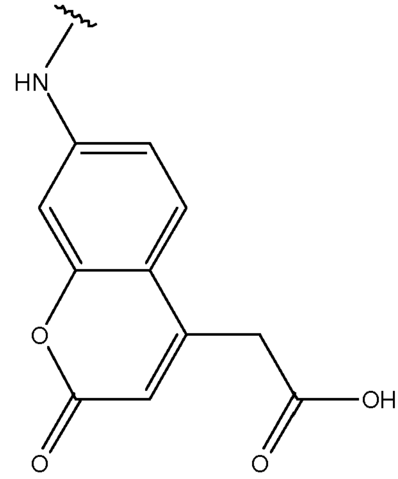

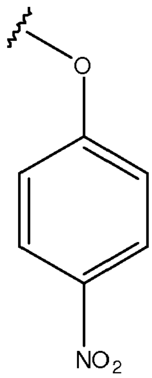

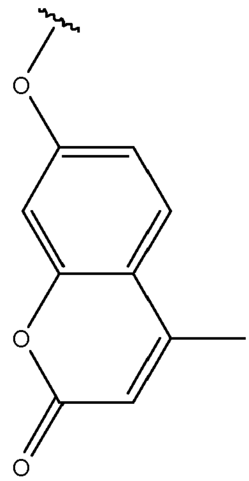

und

- $R_2$ entweder $NH_2$ oder OH ist, und
- A ein mittels Peptidbindung gekoppelter und an seiner α-Amino- oder α-Iminogruppe ungeschützter natürlicher oder unnatürlicher α-Aminosäure- oder α-Iminosäurerest ist, und
- B eine über eine Peptidbindung gekoppelte Peptidsequenz darstellt, die aus 3 bis 13 miteinander über Peptidbindungen gekoppelten natürlichen oder unnatürlichen Aminosäuren oder Iminosäuren besteht.

Als besonders geeignet haben sich Substrate erwiesen, die die beiden folgenden Strukturen als Rest $R_1$ enthalten:

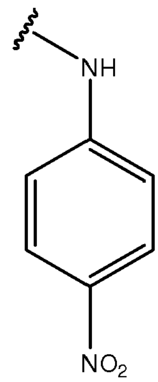 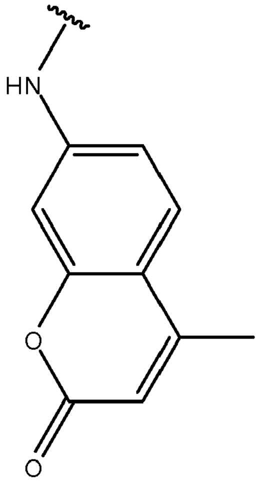

Besonders geeignet haben sich Substrate erwiesen, die nur aus Aminosäureresten in L-Konfiguration aufgebaut sind.

Ganz besonders geeignete Substrate erhält man, wenn man N-terminal als Rest A die aromatischen Aminosäuren Phenylalanin, Tyrosin oder Tryptophan koppelt.

Ebenso haben sich als besonders geeignet Substrate erwiesen, deren Segment B aus insgesamt 4 bis 6 Aminosäureresten besteht.

**Methoden zur Analyse der Verbindungen**

**Analytische HPLC**

Zur analytischen RP-HPLC wurde eine Anlage LC-10A der Firma Shimadzu, bestehend aus den Teilsystemen CTO-10A Säulenofen, LC-10ATvp Pumpen, DGU-14A Degaser, SIL-10Axl Autoinjektor, SCL-10Avp Systemcontroller, SPD-M10Avp Photodiodenarraydetektor und einer Säule 250/4,6 Nucleodur 100-5 C18 ec der Firma Macherey-Nagel (Düren, Deutschland), unter Benutzung der zugehörigen Software Shimadzu CLASS-VP, Version 7.2.1, verwendet. Die Detektion erfolgte bei 220 nm. Als Elutionsmittel dienten Wasser mit 0,1 % TFA (Laufmittel A) und Acetonitril mit 0,1 % TFA (Laufmittel B) bei einer Flussrate von 1 mL/min und einem linearen Gradienten (Beginn bei 10 % Laufmittel B, Anstieg von 1 % B/min).

**Präparative HPLC**

Zur präparativen RP-HPLC wurde eine HPLC-Anlage der Firma Varian, bestehend aus den Teilsystemen Varian PrepStar Modell 218 präparative Pumpen, Varian ProStar Modell 320 UV-Vis Detektor, Varian Fraktionssammler Modell 701, und einer Säule VP 250/32 Nucleodur 100-5 C8 ec der Firma Macherey-Nagel (Düren, Deutschland), unter Benutzung der zugehörigen Star-Software Version 6.0 verwendet. Die Detektion erfolgte bei 220 nm. Als Elutionsmittel dienten ebenfalls Wasser mit 0,1 % TFA (A) und Acetonitril mit 0,1 % TFA (B) bei einer Flussrate von 20 mL/min und einem geeigneten Gradienten.

**Massenspektroskopie**

Die Spektren wurden mit einem Gerät der Firma Applied Biosystems (QTrap 2000) aufgenommen.

**Verwendete Abkürzungen**

| | |
|---|---|
| Ac | Acetyl |
| ACN | Acetonitril |

| | |
|---|---|
| AMC | 7-Amino-5-Methyl-Coumarin bzw. 7-Amido-5-Methyl-Coumarin im Peptid |
| Boc | tert.-Butyloxycarbonyl |
| DIPEA | Diisopropylethylamin |
| DMF | N,N-Dimethylformamid |
| dOD | Änderung der Absorption |
| dRFU | Änderung in den relativen Fluoreszenzeinheiten |
| Fmoc | Fluorenylmethyloxycarbonyl |
| FXIII | inaktive Vorstufe (Zymogen) des Faktors XIII |
| FXIIIa | aktivierter Faktor XIII |
| HBTU | O-Benzotriazol-N,N,N',N'-tetramethyl-uronium-hexafluoro-phosphat |
| i.V. | im Vakuum |
| LM | Lösungsmittel |
| MS | Massenspektroskopie |
| NMP | N-Methylpyrrolidon |
| OSu | Succinimidester |
| Pbf | 2,2,4,6,7-Pentamethyldihydrobenzofuran-5-sulfonyl |
| pNA | p-Nitroanilin bzw. p-Nitroanilid im Peptid gebunden |
| PyBOP | Benzotriazol-1-yl-N-oxy-tris(pyrrolidino)phosphoniumhexafluorophosphat |
| RP-HPLC | reversed-phase Hochleistungs-Flüssigkeitschromatographie |
| RT | Raumtemperatur |
| tBu | tert.-Butyl |
| $t_R$ | Zeit, bei der auf der HPLC die Elution erfolgt (retention time) |
| TFA | Trifluoressigsäure |
| Z | Benzyloxycarbonyl |

Alle eingesetzten Fmoc-Aminosäuren, Harze, Kupplungsreagenzien und anderen Reagenzien für die Synthesen wurden von den Firmen Orpegen, Iris Biotech, Novabiochem, Bachem, Aldrich, Fluka oder Acros bezogen.

**Ausführungsbeispiel 1:**

**Synthese der Ausgangsstoffe**

1a) Fmoc-Glu(pNA)-OH:

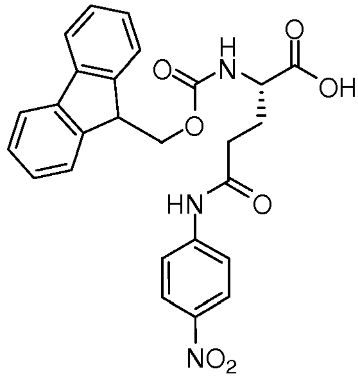

5 g (18,85 mmol) H-Glu(pNA)-OH (Bachem) und 3,28 ml (18,85 mmol) DIPEA werden in 200 ml Wasser und 150 ml ACN suspendiert. Bei 0 °C wird eine Lösung aus 6,36 g (18,85 mmol) Fmoc-OSu in 50 ml ACN über einen Zeitraum von 10 min zugegeben, nach weiteren 10 min werden nochmals 0,6 ml DIPEA hinzugefügt. Der Ansatz wird 30 min unter Eiskühlung und über Nacht bei Raumtemperatur gerührt. Das LM wird i.V. entfernt, der Rückstand in Essigester aufgenommen und jeweils 3 x mit 5 % $KHSO_4$- und gesättigter NaCl-Lösung gewaschen. Die organische Phase wird mit $NaSO_4$ getrocknet. Danach wird das LM i.V. entfernt und das Produkt als leicht gelblicher Feststoff erhalten, der anschließend aus Essigester umkristallisiert wird.
Ausbeute: 8,2 g
HPLC: 50,45 min
MS: ber. 489,15 gef.: 490,06 $(M+H)^+$ und 488,17 $(M-H)^-$

1b) Fmoc-Glu(AMC)-OH:

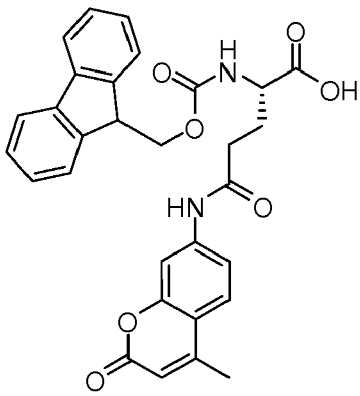

1,627 g (4,468 mmol) H-Glu(AMC)-OH (Sekine et al., Chem. Pharm. Bull. 29, 3286-3289, 1981) und 0,783 ml (4,5 mmol) DIPEA werden in einer Mischung aus 100 ml ACN, 100 ml DMF und 100 ml Wasser suspendiert und bei 0 °C über einen Zeitraum von 10 min mit einer Lösung aus 1,507 g (4,468 mmol) Fmoc-OSu in 20 ml ACN versetzt. Nach 30 min wird das Eisbad entfernt und weitere 0,35 ml DIPEA zugesetzt. Anschließend wird der Ansatz über Nacht bei RT gerührt. Das LM wird i.V. entfernt, der Rückstand in Essigester aufgenommen und jeweils 3 x mit 5 % $KHSO_4$- und gesättigter NaCl-Lösung gewaschen. Die organische Phase wird mit $NaSO_4$ getrocknet, danach wird das LM i.V. entfernt und das Produkt als gelblicher Feststoff erhalten.
Ausbeute: 2,27 g
HPLC: 47,33 min
MS: ber. 526,17 gef.: 527,11 $(M+H)^+$ und 525,21 $(M-H)^-$

**Ausführungsbeispiel 2:**

**Festphasenpeptidsynthese**

Die Festphasenpeptidsynthese wurde mit einem multiplen Syntheseautomat Syro-2000 (MultiSynTech, Witten, Deutschland) in 2 ml Reaktionsgefäßen an Fmoc-Rink-Amid-Polystyren-Harz (100-200 mesh, Ausgangsbeladung 0,63 mmol/g) nach einem Standard-Fmoc-Protokoll durchgeführt. Neben den Standard Fmoc-Aminosäuren ohne funktionelle Gruppe in der Seitenkette wurden folgende seitenkettengeschützte Fmoc-Aminosäuen eingesetzt: Fmoc-Arg(Pbf)-OH, Fmoc-Lys(Boc)-OH, Fmoc-Tyr(tBu)-OH und Fmoc-Ser(tBu)-OH sowie Fmoc-Glu(pNA)-OH und Fmoc-Glu(AMC)-OH. Für die Abspaltung der Fmoc-Schutzgruppe (5 und 20 min) wurde eine Lösung aus Piperidin/DMF/NMP (1/1/1, v/v/v) verwendet, danach wurde das Harz 7 x mit DMF gewaschen. Die Anknüpfung der jeweils folgenden Fmoc-Aminosäure erfolgte durch Doppelkopplung (2x1,5 h), dafür wurden jeweils ca. 4-fache Überschüsse an Fmoc-Aminosäure und Kopplungsreagenz (HBTU) sowie 8-fache Überschüsse an DIPEA als Hilfsbase eingesetzt. Nach Beendigung der Festphasenpeptidsynthese wurde das Harz mehrfach mit DMF, DCM und Methanol gewaschen und i.V. getrocknet. Die Abspaltung vom Harz und von sämtlichen Schutzgruppen erfolgte mit einer Lösung aus 95 % TFA, 2,5 % Triisopropylsilan und 2,5 % Wasser (v/v/v) über einen Zeitraum von 2 h bei Raumtemperatur. Danach wurde die Abspaltlösung aus den Reaktionsgefäßen in Zentrifugenröhrchen mit Diethylether überführt. Das ausgefallene Peptid wurde abzentrifugiert. Die Peptide wurden noch 2 x mit Diethylether gewaschen und mittels präparativer RP-HPLC gereinigt.

Beispielsweise wurden die folgenden Substrate bzw. Verbindungen entsprechend der allgemeinen Formel I auf diese Weise hergestellt (Tabelle 1).

Tabelle 1: Sequenzen der hergestellten pNA- und AMC-Substrate entsprechend der allgemeinen Formel I und deren analytische Charakterisierung.

| Nr. | Sequenz | HPLC $t_R$ (min) | MS-ber. | MS gefund. |
|---|---|---|---|---|
| | pNA-Substrate: | | | |
| S1 | H-Asn-Glu (pNA)-Glu-Asn-Val-Ser-Pro-Leu-$NH_2$ | 24,79 | 1019,47 | 1020,42 |
| S2 | H-Gly-Glu (pNA)-Ser-Lys-Val-Ile-Gly-$NH_2$ | 20,6 | 807,42 | 404,84* |
| S3 | H-Pro-Glu (pNA)-Lys-Lys-Val-Ile-Gly-$NH_2$ | 19,84 | 888,52 | 445,40* |
| S4 | H-Tyr-Glu (pNA)-Ser-Lys-Val-Ile-Gly-$NH_2$ | 22,1 | 913,47 | 457,88* |
| S5 | H-Ile-Glu(pNA)-Val-Lys-Val-Ile-Gly-$NH_2$ | 25,2 | 875,52 | 438,86* |
| S6 | H-Ala-Glu (pNA)-Val-Lys-Val-Ile-Gly-$NH_2$ | 23,7 | 833,48 | 417,86* |
| S7 | H-Phe-Glu (pNA)-Val-Lys-Val-Ile-Gly-$NH_2$ | 27,5 | 909,51 | 455,93* |
| S8 | H-dPhe-Glu (pNA)-Val-Lys-Val-Ile-Gly-$NH_2$ | 28,6 | 909,51 | 910,36 |
| S9 | H-Tyr-Glu (pNA)-Lys-Lys-Val-Ile-Gly-$NH_2$ | 20,33 | 954,52 | 478,34* |
| S10 | H-Tyr-Glu (pNA)-Val-Lys-Val-Ile-Gly-$NH_2$ | 25,09 | 925,5 | 463,89* |

(fortgesetzt)

| Nr. | Sequenz | HPLC $t_R$ (min) | MS-ber. | MS gefund. |
|---|---|---|---|---|
| S11 | H-Tyr-Glu (pNA)-Lys-Val-Val-Ile-Gly-$NH_2$ | 25,3 | 925,5 | 463,95* |
| S12 | H-Tyr-Glu (pNA)-Val-Lys-Val-Ile-$NH_2$ | 26.3 | 868,48 | 435,39* |
| S13 | H-Tyr-Glu (pNA)-Val-Lys-Val-$NH_2$ | 22,9 | 755,4 | 378,83* |
| S14 | Tyr-Glu(pNA)-Ile-Lys-Val-Ile-Gly-$NH_2$ | 26,86 | 939,52 | 940,32 |
| S15 | H-Tyr-Glu (pNA)-Leu-Lys-Val-Ile-Gly-$NH_2$ | 27,24 | 939,52 | 470,75* |
| S16 | H-Tyr-Glu (pNA)-Val-Arg-Val-Ile-Gly-$NH_2$ | 25,47 | 953,51 | 477,77* |
| S17 | H-Tyr-Glu (pNA)-Val-Lys-Val-Phe-$NH_2$ | 28,0 | 902,47 | 903,31 |
| S18 | H-Lys-Glu (pNA)-Val-Lys-Val-Ile-Gly-$NH_2$ | 21,68 | 890,53 | 446,63* |
| S19 | H-His-Glu (pNA)-Lys(Z)-Lys-Val-Ile-Gly-$NH_2$ | 19,25 | 1062,56 | 532,46* |
| S20 | H-Tyr-Glu (pNA)-Lys(Z)-Lys-Val-Ile-Gly-$NH_2$ | 31,85 | 1088,57 | 545,48* |
| | AMC-Substrate: | | | |
| S21 | H-Tyr-Glu (AMC)-Val-Lys-Val-Ile-Gly-$NH_2$ | 33,83 | 962,52 | 963,55 |
| S22 | H-Tyr-Glu (AMC)-Val-Arg-Val-Ile-Gly-$NH_2$ | 34,54 | 990,53 | 991,45 |

*Diese mittels Massenspektrometrie bestimmten Massen sind als als $[M+2H]^{2+}/2$ angegeben, alle anderen gefundenen Massen als $[M+H]^+$.

**Ausführungsbeispiel 3:**

**Messung der Substratspaltung mit pNA-Substraten**

Aliquotierung des Zymogens Faktor XIII:

250 Einheiten lyophilisiertes Fibrogammin HS (Aventis Behring) wurden in 4 ml Wasser gelöst, als 100 µl Portionen aliquotiert und bei -80 °C gelagert. 100 µl dieser Lösung enthalten 6,25 Einheiten FXIII.

Aktivierung des Zymogens FXIII zu Faktor XIIIa:

100 µl des aliqotierten Fibrogammins (entspricht 6,25 Einheiten) wurden mit 500 µl TBS-Puffer (50 mM Tris-HCl, pH 7,5 in 0,9% (w/v) NaCl), 360 µl einer 0.9 % NaCl-Lösung, 10 µl einer 1 M $CaCl_2$-Lösung und 20 µl einer

Thrombinlösung (Rinderthrombin: 14,2 U in 20 µl) versetzt. Der Ansatz wurde 20 min bei 37 °C inkubiert, danach wurden 10 µl einer Lösung mit rekombinantem Hirudin zugesetzt (r-Hirudin: HBW 023 mit 13442 IU $mg^{-1}$, Stammlösung des r-Hirudin = 1 mg/ml). Dieser Aktivierungsansatz besitzt ein Gesamtvolumen von 1 ml, der aktivierte Faktor XIIIa wurde bei 37 °C aufbewahrt und für die folgenden kinetischen Messungen verwendet.

Messansatz mit pNA-Substraten:

Die Messungen wurden bei 405 nm und 37 °C mit einem Microplate Reader der Firma Labsystems (iEMS Reader MF) mit folgendem Messansatz durchgeführt:

- 150 µl TBS-Puffer (siehe oben, der Puffer kann gegebenenfalls auch eine Aminkomponente enthalten, beispielsweise H-Gly-Ethylester oder H-Gly-Gly-Ethylester oder ein anderes Amin, falls vorhanden ist die finale Konzentration des Amins im Messansatz 2,5 mM oder höher)
- 50 µl Substratlösung
- 50 µl Enzymlösung (im Messansatz ca. 1,25 Einheiten/ml)

Durchführung der Messung:

Sämtliche Lösungen wurden vor Benutzung bei 37 °C aufbewahrt, vor Zugabe der FXIIIa-Lösung wurde die Mikroreadermessplatte nochmals 5 min bei 37 °C im Messgerät temperiert. Nach Zugabe der FXIIIa-Lösung wurde die Substratspaltung gestartet und die Änderung der Absorption bei 405 nm gemessen. Für einen Vortest wurde eine konstante Substratkonzentration verwendet. Dabei wurde gefunden, dass beispielsweise das Substrat S1, dass sich von der N-terminalen Sequenz des FXIIIa-Substrates $\alpha_2$-Antiplasmin ableitet (Cleary et al., Biochimica et Biophysica Acta 1764, 1207-1217 (2006)) praktisch kaum gespalten wird, auch das Substrat S2 mit N-terminalem Glycinrest, abgeleitet von dem im Berichromtest verwendeten Substrat, wird nur langsam umgesetzt. Wir haben vermutet, dass vor allem die Aminosäuren N- und C-terminal des Glu(pNA)-Restes wichtig für die Substraterkennung sind, deshalb wurde das N-terminale Glycin und das an dritter Position befindliche Serin innerhalb der Berichromsequenz durch andere Aminosäurereste ersetzt. Durch den Vortest wurde festgestellt, dass einige dieser Substrate deutlich schneller als die Derivate S1 und S2 gespalten werden. Für ausgewählte Substrate wurden die Substratkonzentrationen im Messansatz variiert und die bestimmten Umsatzgeschwindigkeiten als Funktion der Substratkonzentration entsprechend der Michaelis-Menten-Gleichung $v=V_{max}\cdot[S]/(K_m+[S])$ aufgetragen und somit die kinetischen Konstanten $K_m$ und $V_{max}$ bestimmt, wobei $V_{max}$ in der Einheit Absorptionsänderung bei 405 nm/s erhalten wurde. Dies ist in Fig. 1 und Fig. 2 gezeigt.

Als geeignete Substrate haben sich unter anderem auch die Verbindungen S14 und S16 erwiesen (Fig. 2).

**Ausführungsbeispiel 4:**

**Messung der Substratspaltung mit AMC-Substraten**

Die Aliquotierung des Zymogens Faktor XIII und Aktivierung des Zymogens FXIII zu Faktor XIIIa erfolgten analog der in Beispiel 3 beschriebenen Prozedur.

Messansatz mit AMC-Substraten:

Sämtliche Lösungen wurden bei Raumtemperatur aufbewahrt. Die Bestimmung der Spaltung der AMC-Substrate durch FXIIIa wurde mit einem Fluoreszenz-Plattenreader Safire[2] der Firma Tecan ($\lambda_{EX}$ = 380 nm, $\lambda_{Em}$ = 460 nm) bei Raumtemperatur durchgeführt.

- 100 µl TBS-Puffer (siehe oben, der Puffer kann gegebenenfalls auch eine Aminkomponente enthalten, beispielsweise H-Gly-Ethylester oder H-Gly-Gly-Ethylester oder ein anderes Amin)
- 50 µl Substratlösung
- 25 µl Enzymlösung (im Messansatz ca. 0,893 Einheiten/ml)

Die Michaelis-Menten-Auftragung für die AMC-enthaltenden Substrate ist in Fig. 3 gezeigt.

**Abbildungslegenden**

**Fig. 1**

Michaelis-Menten-Auftragung für die FXIIIa-katalysierte Spaltung der Peptidsubstrate S10 (o) und S12 (•). Für diese beiden pNA-Substrate wurden folgende kinetischen Konstanten bestimmt:

$$S10: K_m = 40\ \mu M, \quad V_{max}: 0{,}95 \times 10^{-4}\ dOD/s$$

$$S12: K_m = 86\ \mu M, \quad V_{max}: 1{,}33 \times 10^{-4}\ dOD/s$$

**Fig. 2**

Michaelis-Menten-Auftragung für die FXIIIa-katalysierte Spaltung der Peptidsubstrate S16 (o) und S14 (•). Für diese beiden pNA-Substrate wurden folgende kinetischen Konstanten bestimmt:

$$S14: K_m = 78\ \mu M, \quad V_{max}: 1{,}2 \times 10^{-4}\ dOD/s$$

$$S16: K_m = 49\ \mu M, \quad V_{max}: 0{,}8 \times 10^{-4}\ dOD/s$$

**Fig. 3**

Michaelis-Menten-Auftragung für die FXIIIa-katalysierte Spaltung der Peptidsubstrate S21 (•) und S22 (•). Für die beiden AMC-Substrate wurden folgende kinetischen Konstanten bestimmt:

$$S18: K_m = 30\ \mu M, \quad V_{max}: 9{,}61\ dRFU/s$$

$$S19: K_m = 41\ \mu M, \quad V_{max}: 11{,}99\ dRFU/s$$

## Patentansprüche

**1.** Spektroskopisches Verfahren zur UV/VIS- oder fluoreszenzbasierenden kinetischen Bestimmung der Aktivität von Transglutaminasen, speziell der Transglutaminase Faktor XIIIa, **dadurch gekennzeichnet, dass** Faktor XIIIa ein modifiziertes Peptidsubstrat gemäß der allgemeinen Formel (I)

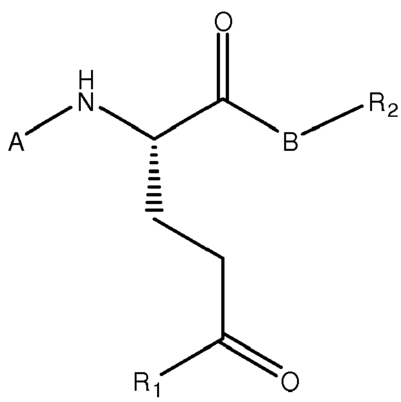

(I)

spaltet und die resultierende Änderung der UV/Vis-Ab oder der Fluoreszenz bei geeigneten Wellenlängen bestimmt wird, in dem

- $R_1$ eine der folgenden Strukturen ist

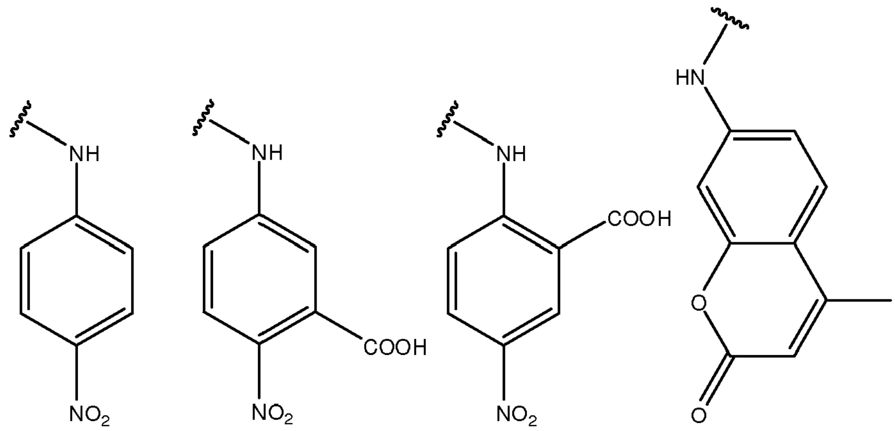

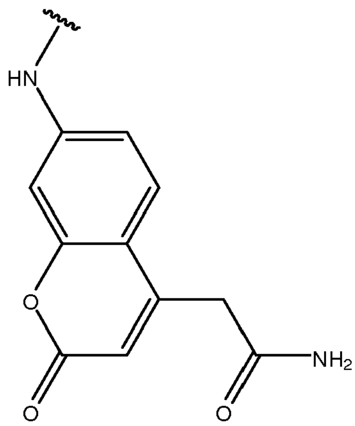

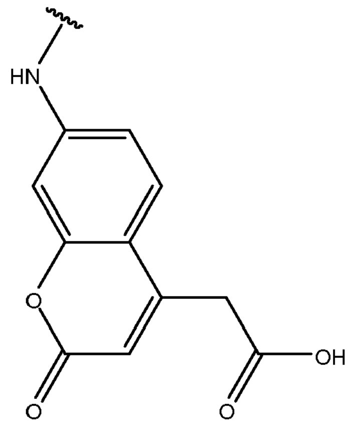

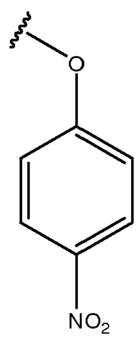

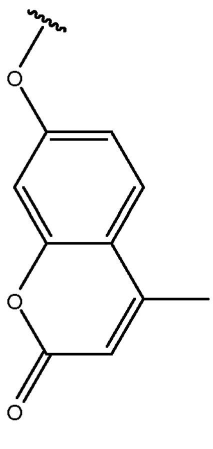

und

- $R_2$ entweder $NH_2$ oder OH ist, und
- A ein mittels Peptidbindung gekoppelter beliebiger an der α-Amino- oder Iminogruppe ungeschützter natürlicher oder unnatürlicher α-Aminosäure- oder α-Iminosäurerest ist, und
- B eine über eine Peptidbindung gekoppelte Peptidsequenz darstellt, die aus 3 bis 13 miteinander über Peptidbindungen gekoppelten natürlichen oder unnatürlichen Aminosäuren oder Iminosäuren besteht.

2. Verfahren nach Anspruch 1, **dadurch gekennzeichnet, dass** $R_1$ in der allgemeinen Formel I eine der folgenden Strukturen ist

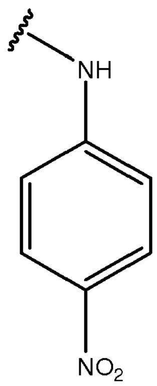 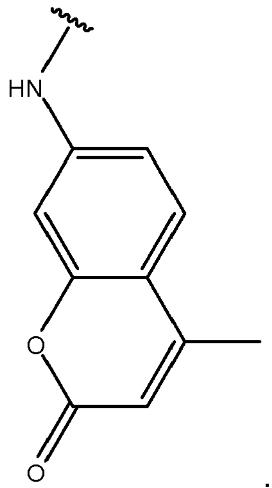 .

3. Verfahren nach Anspruch 1 und 2, **dadurch gekennzeichnet, dass** A in der allgemeinen Formel I ein beliebiger an der α-Aminogruppe ungeschützter natürlicher oder unnatürlicher α-Aminosäurerest in L-Konfiguration ist, bevorzugt handelt es sich um einen gekoppelten Tyrosin-, Phenylalanin-, Tryptophan- oder Cyclohexylalaninrest.

4. Verfahren nach Anspruch 1 bis 3, **dadurch gekennzeichnet, dass** B in der allgemeinen Formel I eine Peptidsequenz darstellt, die aus 3 bis 8 miteinander über Peptidbindungen gekoppelten natürlichen oder unnatürlichen α-Aminosäuren oder α-Iminosäuren besteht, bevorzugt besteht die Peptidsequenz aus 4-6 Resten und ganz besonders bevorzugt aus 4 bis 5 Resten.

5. Verfahren nach Anspruch 1 bis 4, **dadurch gekennzeichnet, dass** B in der allgemeinen Formel I eine Peptidsequenz darstellt, die nur aus α-Amino- oder α-Iminosäuren in der L-Konfiguration besteht.

6. Verfahren nach Anspruch 1 bis 5, **dadurch gekennzeichnet, dass** H-Gly-Pro-Arg-Pro-$NH_2$ oder ein ähnliches Peptidderivat als Fibrinaggregationshemmer eingesetzt wird und der Faktor XIIIa aus dem Zymogen Faktor XIII durch Thrombin aktiviert wird.

7. Verfahren nach Anspruch 1 bis 6, **dadurch gekennzeichnet, dass** ein Amin zugesetzt wird, bevorzugt ist das Amin ein Glycinmethyl- oder ethylester, ein Glycyl-Glycinmethyl- oder ethylester, Ethanolamin, Diaminoethan, Diaminopropan oder ein Derivat des 1,5-Diaminopentans.

8. Verfahren nach Anspruch 1-7, **dadurch gekennzeichnet, dass** der Faktors XIIIa in gereinigter oder auch in ungereinigter Form, z.B. in Zellhomogenisaten, in Plasma, in Serum oder in Blut gemessen werden kann.

9. Verfahren nach Anspruch 1 bis 8, **dadurch gekennzeichnet, dass** die Aktivitätsmessung sowohl als Einzelmessung, als auch als parallele Messung in einer für ein parallelisiertes Messverfahren geeigneten Vorrichtung durchgeführt werden kann, beispielsweise auch in einer Mikotiterplatte oder in Screeningassays.

10. Verfahren zur Identifizierung von Verbindungen, die eine Faktor XIIIa-Aktivität modulieren, umfassend das Inkontaktbringen von wenigstens einer Verbindung mit Faktor XIIIa und anschließender Messung der Aktivität des Faktors XIIIa gemäß einem der Ansprüche 1 bis 9, besonders zur Identifizierung von Verbindungen, die die Aktivität des Faktors XIIIa verstärken oder verringern, ganz besonders zur Identifizierung von Hemmstoffen des Faktors XIIIa.

11. Peptidsubstrat für Faktor XIIIa nach der allgemeinen Formel I, **dadurch gekennzeichnet, dass** es aus folgenden

Strukturen ausgewählt wird:

X-Glu(pNA)-Y-Z-Y-Y-T oder
X-Glu(AMC)-Y-Z-Y-Y-T, indem

X ausgewählt wird aus den Aminosäureresten Phe, Tyr, Trp und indem
Y unabhängig voneinander ausgewählt wird aus den Aminosäureresten Ile, Leu, Val, Phe, Tyr, Lys, Arg und indem
Z ausgewählt wird aus den Aminosäureresten Lys oder Arg, und indem
T ausgewählt wird aus -OH, $-NH_2$, Ala-OH, Ala-$NH_2$, Phe-OH, Phe-$NH_2$, Gly-OH oder Gly-$NH_2$,

besonders von folgenden Strukturen:

H-Tyr-Glu(pNA)-Ser-Lys-Val-Ile-Gly-$NH_2$
H-Ile-Glu(pNA)-Val-Lys-Val-Ile-Gly-$NH_2$
H-Ala-Glu(pNA)-Val-Lys-Val-Ile-Gly-$NH_2$
H-Phe-Glu(pNA)-Val-Lys-Val-Ile-Gly-$NH_2$
H-Tyr-Glu(pNA)-Lys-Lys-Val-Ile-Gly-$NH_2$
H-Tyr-Glu(pNA)-Val-Lys-Val-Ile-Gly-$NH_2$
H-Tyr-Glu(pNA)-Lys-Val-Val-Ile-Gly-$NH_2$
H-Tyr-Glu(pNA)-Val-Lys-Val-Ile-$NH_2$
Tyr-Glu(pNA)-Ile-Lys-Val-Ile-Gly-$NH_2$
H-Tyr-Glu(pNA)-Leu-Lys-Val-Ile-Gly-$NH_2$
H-Tyr-Glu(pNA)-Val-Arg-Val-Ile-Gly-$NH_2$
H-Tyr-Glu(pNA)-Val-Lys-Val-Phe-$NH_2$
H-Lys-Glu(pNA)-Val-Lys-Val-Ile-Gly-$NH_2$,

wobei in allen Substraten der p-Nitroanilid-Rest auch durch einen Amidomethylcoumarin-Rest ausgetauscht sein kann.

**12.** Verwendung eines Peptidsubtrates gemäß Ansprüchen 1 bis 11 in einem Verfahren zur UV/VIS- oder fluoreszenzbasierten kinetischen Messung der Faktor XIIIa-Aktivität.

Fig. 1

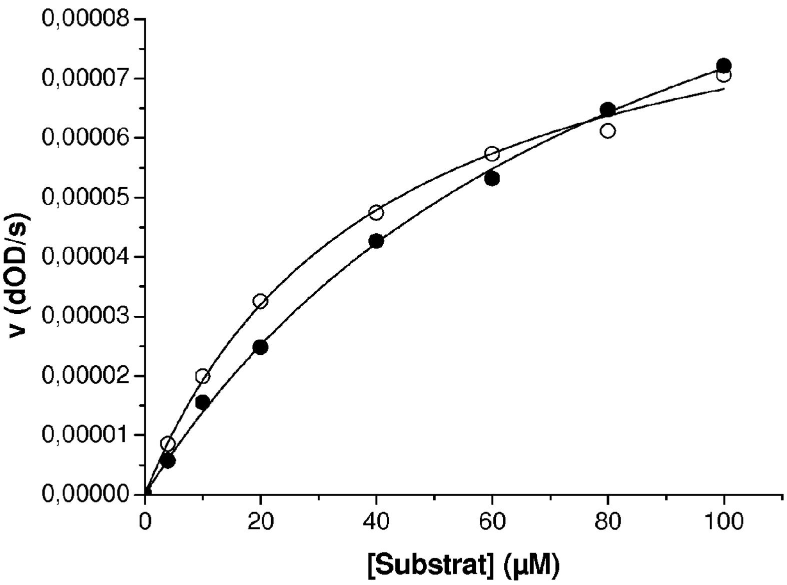
0,00008
0,00007
0,00006
0,00005
0,00004
0,00003
0,00002
0,00001
0,00000
v (dOD/s)
0
20
40
60
80
100
[Substrat] (µM)

Fig. 2

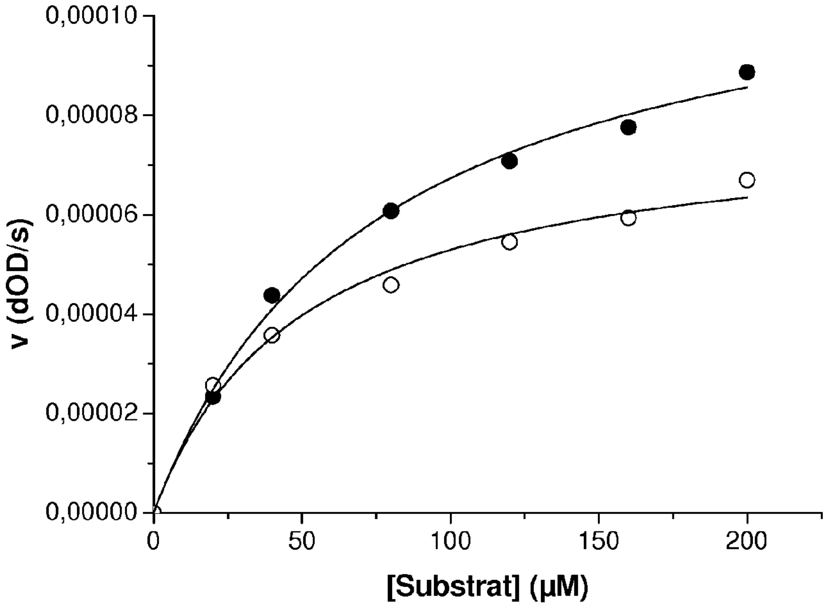
0,00010
0,00008
0,00006
0,00004
0,00002
0,00000
v (dOD/s)
0
50
100
150
200
[Substrat] (µM)

Fig. 3

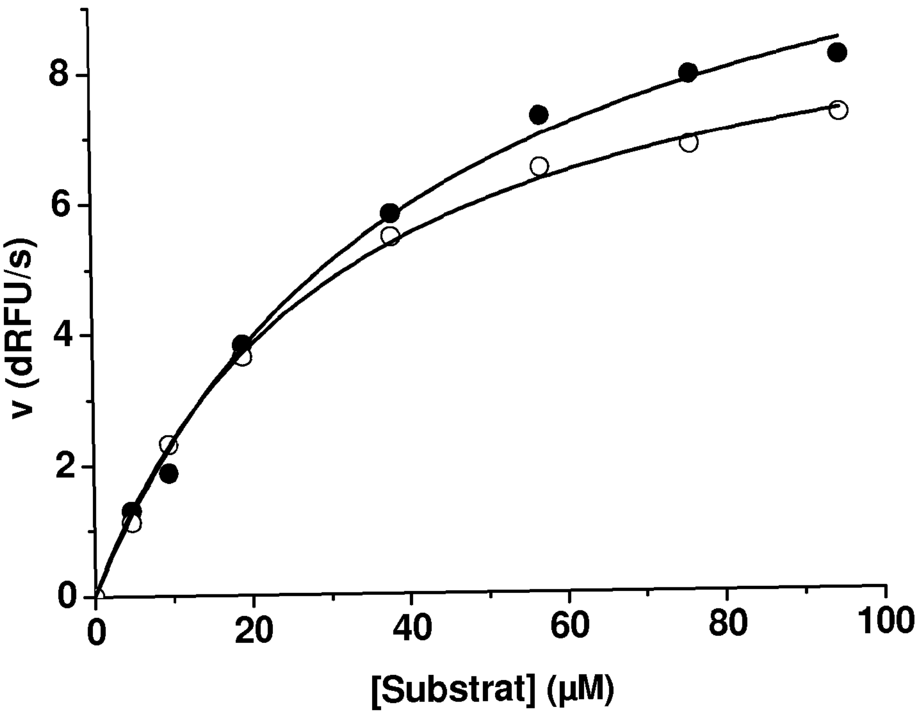

Europäisches Patentamt
European Patent Office
Office européen des brevets

# EUROPÄISCHER RECHERCHENBERICHT

Nummer der Anmeldung
EP 10 15 1487

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | OERTEL ET AL: "A highly sensitive fluorometric assay for determination of human coagulation factor XIII in plasma" ANALYTICAL BIOCHEMISTRY, ACADEMIC PRESS INC, NEW YORK LNKD- DOI:10.1016/J.AB.2007.05.011, Bd. 367, Nr. 2, 3. Juli 2007 (2007-07-03), Seiten 152-158, XP022138785 ISSN: 0003-2697 * das ganze Dokument, insbesondere Abb. 1 * | 1-12 | INV. C07K7/06 C12Q1/52 G01N33/58 |
| A | DE MACEDO PIERRE ET AL: "A direct continuous spectrophotometric assay for transglutaminase activity" ANALYTICAL BIOCHEMISTRY, Bd. 285, Nr. 1, 1. Oktober 2000 (2000-10-01), Seiten 16-20, XP002582110 ISSN: 0003-2697 * das ganze Dokument * | 1-12 | |
| | | | RECHERCHIERTE SACHGEBIETE (IPC) |
| A | SMITH G D ET AL: "A sensitive fluorimetric assay for gamma-glutamyl transferase" ANALYTICAL BIOCHEMISTRY, ACADEMIC PRESS INC, NEW YORK LNKD- DOI:10.1016/0003-2697(79)90122-2, Bd. 100, Nr. 1, 15. November 1979 (1979-11-15), Seiten 136-139, XP024822209 ISSN: 0003-2697 [gefunden am 1979-11-15] * das ganze Dokument * | 1-12 | C07K C12Q G01N |
| A | EP 1 361 439 A1 (PASTEUR INSTITUT [FR]) 12. November 2003 (2003-11-12) * das ganze Dokument * | 11 | |

Der vorliegende Recherchenbericht wurde für alle Patentansprüche erstellt

2

| Recherchenort | Abschlußdatum der Recherche | Prüfer |
|---|---|---|
| München | 17. Mai 2010 | Lüdemann, Susanna |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 10 15 1487

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-05-2010

| Im Recherchenbericht angeführtes Patentdokument | Datum der Veröffentlichung | Mitglied(er) der Patentfamilie | Datum der Veröffentlichung |
|---|---|---|---|
| EP 1361439 A1 | 12-11-2003 | AT 345504 T | 15-12-2006 |
| | | AT 382864 T | 15-01-2008 |
| | | AT 434186 T | 15-07-2009 |
| | | AU 2003242569 A1 | 11-11-2003 |
| | | CA 2484211 A1 | 20-11-2003 |
| | | DE 60216048 T2 | 31-05-2007 |
| | | DE 60318427 T2 | 18-12-2008 |
| | | DK 1361439 T3 | 26-03-2007 |
| | | DK 1502116 T3 | 13-05-2008 |
| | | DK 1788394 T3 | 19-10-2009 |
| | | WO 03096022 A2 | 20-11-2003 |
| | | EP 1502116 A2 | 02-02-2005 |
| | | EP 1788394 A1 | 23-05-2007 |
| | | EP 1933150 A1 | 18-06-2008 |
| | | ES 2276895 T3 | 01-07-2007 |
| | | ES 2300603 T3 | 16-06-2008 |
| | | ES 2327773 T3 | 03-11-2009 |
| | | HK 1062198 A1 | 08-06-2007 |
| | | HK 1078337 A1 | 21-11-2008 |
| | | PT 1361439 E | 28-02-2007 |
| | | PT 1502116 E | 15-04-2008 |
| | | PT 1788394 E | 13-08-2009 |
| | | SI 1502116 T1 | 30-06-2008 |
| | | US 2005244844 A1 | 03-11-2005 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **WILMER et al.** *Hämostaseologie,* 2002, vol. 22, 18-28
- **MUSZBEK et al.** *Cardiovascular & Hematological Agents in Medicinal Chemistry,* 2008, vol. 6, 190-205
- **PRASA ; STÜRZEBECHER.** *Hämostaseologie,* 2002, vol. 22, 29-33
- **OERTEL et al.** *Analytical Biochemistry,* 2007, vol. 367, 152-158
- **FICKENSCHER et al.** *Thromb. Haemost.,* 1991, vol. 65, 535-540
- **KARPATI et al.** *Clin. Chem.,* 2000, vol. 46, 1946-1955
- **WODZINSKA.** *Mini Rev. in Med. Chem.,* 2005, vol. 5, 279-292
- **SMITH et al.** *Biochem.,* 1979, vol. 100, 136-139
- **SEKINE et al.** *Chem. Pharm. Bull.,* 1981, vol. 29, 3286-3289
- **CLEARY et al.** *Biochimica et Biophysica Acta,* vol. 1764, 1207-1217